Europäisches Patentamt

European Patent Office

Office européen des brevets

(11) Veröffentlichungsnummer: **0 302 407**
**A2**

## (12) EUROPÄISCHE PATENTANMELDUNG

(21) Anmeldenummer: 88112339.2

(51) Int. Cl.⁴: **C07K 7/06 , C12P 21/04 ,**
**A23K 1/17**

(22) Anmeldetag: 29.07.88

Patentansprüche für folgende Vertragsstaaten:ES
und GR

(30) Priorität: 05.08.87 DE 3725940

(43) Veröffentlichungstag der Anmeldung:
08.02.89 Patentblatt 89/06

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Dürckheimer, Walter, Dr.
Im Lerchenfeld 45
D-6234 Hattersheim am Main(DE)
Erfinder: Seliger, Hubert
Kallestrasse 3
D-6000 Frankfurt am Main(DE)
Erfinder: Dost, Günter, Dr.
Im Hain 6
D-6233 Kelkheim (Taunus)(DE)
Erfinder: Chatterjee, Sugata
H1/2 Hoechst Quarters Darga Road
Mulund (West) Bombay 400 082(IN)
Erfinder: Desikan, Kalyanapuram Rajagopalan
4, Krishna Kunj Opp. Johnson L.B.S. Marg
Mulund (West) Bombay 400 080(IN)
Erfinder: Rupp, Richard Helmut, Dr.
Roederweg 16a
D-6240 Königstein/Taunus(DE)
Erfinder: Ganguli, Bimal Naresh, Dr.
Saurayuth 173 Central Avenue
Chembur Bombay 400 071(IN)
Erfinder: Franco, Christopher Milton Mathew,
Dr.
74 A, The Westminister N.S. Mankikar Marg.
Sion Bombay 400 022(IN)

(54) Streptogramin B-Metallkomplexe, ihre Herstellung und Verwendung in der Tierernährung.

(57) Streptogramin B-Metallkomplexe, insbesondere die Cu-, Zn-und Mn-Komplexe des Etamycins, sind wertvolle Leistungsförderer in der Tierernährung. Man erhält sie durch Umsetzung von Neoviridogriseinen mit Metallsalzen in Lösung und anschließender Neutralisierung mit Basen.

EP 0 302 407 A2

**Streptogramin B-Metallkomplexe, ihre Herstellung und Verwendung in der Tierernährung**

Zu den Antibiotika vom Streptogramin B-Typ gehören Etamycin (auch Viridogrisein oder Neoviridogrisein IV (NVG IV) genannt), ferner Neoviridogrisein I, II und III sowie Grividomycin I, II und III. Sie sind durch die folgende allgemeine Formel charakterisiert:

| | $R_1$ | $R_2$ | $R_3$ | $R_4$ |
|---|---|---|---|---|
| VIRIDOGRISEIN ETAMYCIN, NEOVIRIDOGRISEIN IV | OH | $CH_3$ | $CH_3$ | $CH_3$ |
| VIRIDOGRISEIN II | OH | H | $CH_3$ | $CH_3$ |
| NEOVIRIDOGRISEIN I | H | $CH_3$ | $C_2H_5$ | $CH_3$ |
| NEOVIRIDOGRISEIN II | H | $CH_3$ | $CH_3$ | $CH_3$ |
| NEOVIRIDOGRISEIN III | OH | $CH_3$ | $C_2H_5$ | $CH_3$ |
| GRIVIDOMYCIN I / II | OH | $CH_3$ | H | $CH_3$ |
| GRIVIDOMYCIN III | OH | $CH_3$ | $CH_3$ | H |

(Lit.: Vazquez, Antibiotics III, Mechanism of action of antimicrobial and antitumor agents, S. 521 ff. (1975)). Sie werden - meist als Gemisch mehrerer Antibiotika - bei der Fermentation bestimmter Streptomyces-Arten gebildet. So entsteht durch Züchtung von Streptomyces griseus NRRL 2426 Etamycin neben Griseoviridin (US-PS 3.023.204) und von Streptomyces sp. P 8648 (FERM - P 3562) neben Etamycin die NVGs I-III (US-PS 4.536.397). Schließlich werden gemäß der europäischen Anmeldung 87 101 796.8 bei der Fermentation von Streptomyces sp. Y-82 40 155 (DSM 3640) außer Etamycin unter anderem noch die Grividomycine I, II und III gebildet.

Die Streptogramin B-Antibiotika sind gegen gram-positive Bakterien und Mycoplasmen wirksam und finden z.T. Anwendung in der Veterinärmedizin und als Futterzusatzstoffe zur Verbesserung der Gewichtszunahme bei Nutztieren (US-PS 4.355.122 und 4.536.397).

Es wurde nun gefunden, daß Metallkomplexe der genannten Verbindungen in Mischung mit Futter bereits in sehr kleinen Mengen zu einer hoch-signifikanten und gleichmäßigen Gewichtszunahme der Tiere führen, die höher ist als sie bei Verabreichung der freien Antibiotika erzielt wird.

Gegenstand der vorliegenden Erfindung sind somit Metallkomplexe von Streptogramin B-Antibiotika, ihre Herstellung und ihre Verwendung als wachstumssteigernde und die Nährstoffverwertung verbessernde Wirkstoffe in der Tierernährung.

Die Metallkomplexe enthalten als Zentralatome physiologisch verträgliche zwei- oder dreiwertige Kationen von Metallen, die generell als Spurenelemente in der Tierernährung Verwendung finden, wie Kupfer, Zink, Mangan, Eisen, Kobalt, Magnesium, Kalzium und Aluminium. Das Verhältnis NVG:Metallkation liegt zwischen 1:1 und (je nach der Wertigkeit des Kations) 2:1 oder 3:1. Die allgemeine Formel der Komplexe lautet also

$$a \, [SB] \times Me^{n+} \times (n-a) \, OH \qquad II$$

wobei SB ein Streptogramin B-Antibiotikum, Me das Metallkation, n 2 oder 3 und a eine Zahl von 1 bis n (n $\geq$ a) bedeutet.

Innerhalb des angegebenen Bereichs kann die Zusammensetzung der Komplexe beliebig variieren. Man kann also jeweils diejenige Zusammensetzung wählen, die vom Anwendungsziel her am vorteilhaftesten und geeignetsten erscheint.

Bevorzugt sind die Komplexe des Etamycin der Antibiotika der Formel I, da dieses bei der Fermentation der genannten Streptomyceten das Hauptprodukt bildet. Geeignet sind ferner Gemische von Komplexen zweier oder mehrerer Metalle die stöchiometrisch unterschiedlich zusammengesetzt sein können, z.B. Gemische aus den Komplexen von Kupfer/Zink, Mangan/Kobalt, Magnesium/Eisen, Kupfer/Mangan/Magnesium usw. Praktische Bedeutung haben auch Gemische von Komplexen verschiedener Streptogramin B-Antibiotika. Dies bietet den Vorteil, daß man die bei der Fermentation erhaltenen Antibiotika-Gemische ohne vorherige Trennung komplexieren kann.

Die Komplexe werden aus den Antibiotika der Formel I und Metallsalzen in Lösung hergestellt. Erstere sind in reiner Form und als Gemische in vielen organischen Lösungsmitteln, wie beispielsweise Methanol, Äthanol, Propanol, i-Propanol, Butanol, Methylenchlorid, Diäthyläther, Tetrahydrofuran, Dioxan, Dimethylformamid, Essigsäureäthylester und Äther löslich. Man versetzt die Lösung der metallfreien Antibiotika (rein oder als Komponentengemisch) unter Umschütteln oder Rühren mit der für die angestrebte Zusammensetzung notwendigen Menge Metallsalzlösung; anschließend setzt man eine anorganische oder organische Base zu, die der Metallsalzmenge äquivalent ist.

Die Metallsalze leiten sich von beliebigen physiologisch verträglichen anorganischen und organischen Säuren ab. Geeignet sind z.B. Chloride, Bromide, Sulfate, Phosphate und Carboxylate organischer Säuren, wie z.B. Formiate, Acetate, Sulfonate, Phenolate und Enolate. Die Salze können sowohl als Hydrate als auch in wasserfreier Form eingesetzt werden. Zur Umsetzung werden sie bei Raumtemperatur in Wasser oder einem polaren organischen Lösungsmittel, wie z.B. Methanol oder in Gemischen von organischen Lösungsmitteln mit Wasser gelöst.

Die Operationen werden vorzugsweise bei Raumtemperatur ausgeführt, können aber auch oberhalb und unterhalb derselben ausgeführt werden, wenn dies die Löslichkeit der Komponenten oder die Flüchtigkeit der Lösungsmittel erfordert. Die Komplexbildung erfolgt sehr rasch und gibt sich bei der Verwendung gefärbter Metallionen wie z.B. Cu(II)-, Co(II)- oder Fe(III)-Ionen auch durch eine augenblickliche Farbänderung bzw. eine Veränderung des UV-und sichtbaren Spektrums zu erkennen.

Durch den Zusatz einer Base werden die bei der Komplexbildung freigesetzten Protonen anschließend neutralisiert. Hierzu können alle organischen und anorganischen Basen eingesetzt werden, die nicht mit den Metallionen selbst stark komplexieren. Unter den anorganischen Basen eignet sich besonders Natronlauge und Kalilauge. Mit dem gleichen Erfolg können aber auch Alkoholate wie Natriummethylat oder organische Basen, vorzugsweise tert. Basen wie Trimethylamin, Triäthylamin, N-Methylpiperidin, Pyridin, Collidin und Diisopropyl-äthylamin verwendet werden.

Man isoliert die Komplexe, indem man die Reaktionslösung bei Raumtemperatur im Vakuum oder bei Normaldruck einengt. Aus dem Rückstand werden nicht komplexierte Salze und wasserlösliche Verunreinigungen mit Wasser ausgewaschen. Die abgefilterten Metallkomplexe können gegebenenfalls nach dem Trocknen mit wenig polaren organischen Lösungsmitteln gewaschen werden. Dadurch lassen sich nicht komplexierte Anteile der Neoviridogriseine oder eventuell vorliegende organische Verunreinigungen entfernen. Als organische Lösungsmittel eignen sich Äther wie Diäthyl-. Di-i-propyl-, Dipropyl- und Dibutyläther.

Die erfindungsgemäßen Metallkomplexe sind überraschend stabile Verbindungen. Sie verändern sich nicht an der Luft oder am Tageslicht und sind nicht hygroskopisch. In Wasser sind sie sehr schwer löslich. Sie lösen sich im Gegensatz zu den freien Antibiotika auch schwer in wenig polaren oder unpolaren organischen Lösungsmitteln wie Äther; leichter dagegen in Alkoholen. Sie besitzen höhere Zersetzungspunkte und können stärker thermisch belastet werden als die nicht komplexierten Antibiotika. Sie lassen sich aufgrund ihrer physikalischen Eigenschaften gut verteilen und mit anderen Futterzusatzstoffen mischen.

Aufgrund ihrer antibiotischen Eigenschaften, sehr guten Verträglichkeit und ihrer physikochemischen Eigenschaften sind die erfindungsgemäßen Komplexe als wachstumsfördernde Mittel in der Tierernährung sehr geeignet, besonders bei der Fütterung und Aufzucht von Geflügel (Masthühner, Legehennen, Puten), Ferkeln und Mastschweinen sowie Mastrindern und Lämmern. Sie können mit beliebigen anderen Futterzusatzstoffen (z.B. antimikrobiellen Wirkstoffen, anderen Antibiotika, inerten Füllstoffen wie Kalziumcarbonat oder Kieselgel sowie Tierfutter der verschiedensten Zusammensetzungen) zusammen angewendet werden. Besonders erwähnt seien Mischungen der Komplexe mit den ihnen zugrundeliegenden komplexfreien Antibiotika, die entweder durch einfaches Vermischen erzeugt werden können oder dadurch, daß man bei der Komplexierung das Metallsalz im stöchiometrischen Unterschuß anwendet.

Eine bevorzugte Applikationsform ist die Zugabe zum Futter in Form eines Konzentrates (Praemix). Das Konzentrat kann beispielsweise durch Vermischen des Wirkstoffes, des Rohproduktes oder des wirkstoffhaltigen Mycels mit einem physiologisch verträglichen, festen oder flüssigen Träger hergestellt werden. Als fester Träger kommen beispielsweise Getreidenebenprodukte wie Weizennachmehl, Weizenkleie oder entölte Reiskleie, aber auch Maismehl, Sojamehl, Bolus alba oder Kalziumkarbonat sowie Zellulosederivate wie Methyl-und Carboxymethylzellulose in Betracht. Als flüssige Träger können physiologische Salzlösungen, destilliertes Wasser und physiologisch verträgliche organische Lösungsmittel Verwendung finden. Es ist auch möglich, geeignete Zusatzstoffe, wie Emulgatoren, Dispersionsmittel, Suspensionsmittel, Benetzungsmittel oder Geliermittel zuzusetzen. Diese Konzentrate können in der Regel etwa 0,5 bis etwa 5 % des Wirkstoffs enthalten, wobei je nach Anwendungszweck die Wirkstoffkonzentration auch erheblich über- oder unterschritten werden kann.

Bei der Verabreichung mit Futtermitteln wird zweckmäßigerweise so verfahren, daß ein Konzentrat mit dem Futter vermengt wird, beispielsweise durch Einmischen, Vermahlen, Schütteln und Einrühren.

Eine andere Form der Verabreichung besteht darin, daß der Wirkstoff als solcher oder in Form eines Konzentrates oder suspendierbaren Pulvers dem Trinkwasser oder einem anderen Getränk, wie Milchaustauschfutter, beigegeben wird.

Die Dosierung kann je nach Indikation und Applikationsart etwa 0,02 bis etwa 5,0, vorzugsweise etwa 0,2 bis etwa 2,0 mg Wirkstoff pro kg Körpergewicht und Tag betragen. Im Futter resultieren daraus Konzentrationen von etwa 0,5 - 500, vorzugsweise etwa 2 - 100 g Wirkstoff/Tonne.


**Herstellungsbeispiele**


1) Etamycin-Kupfer-(II)-komplex (Et) x Cu(II)OH

879 mg (1 mMol) Etamycin gelöst in 10 ml Methanol werden mit einer Lösung von 170,5 mg (1 mMol) Kupfer(II)-chlorid-dihydrat in 5 ml Methanol versetzt. Unter Rühren läßt man 2 ml 1 n methanolische NaOH hinzutropfen. Man destilliert im Vakuum das Methanol ab, verreibt den Rückstand mit Wasser, saugt ab und wäscht mit Wasser, bis das durchlaufende Filtrat frei von Cl-Ionen ist. Anschließend wird der Kupferkomplex in Äther suspendiert. Man rührt 30 Minuten, saugt ab, wäscht mit Äther nach und trocknet im Vakuum über $P_2O_5$. Ausbeute: 907 mg Etamycin-Kupferkomplex.

| $C_{44}H_{62}CuN_8O_{12}$ (958,54) | | | | | | |
|---|---|---|---|---|---|---|
| ber. | C | 54,0 % | H | 6,6 % | Cu | 6,5 % |
| gef. | | 54,8 % | | 6,7 % | | 6,0 % |

Die berechneten Analysenwerte sind auf einen Wassergehalt (bestimmt nach Karl Fischer) von 2,0 % bezogen.

4

$\lambda_{max.}$ (CH$_3$OH) = 340 nm

## 2) Etamycin-Kupfer-(II)-komplex (Et$_2$ x Cu(II))

Zu einer Lösung von 2,64 g (3 mMol) Etamycin in 30 ml Methanol wird eine Lösung von 256 mg (1,5 mMol) Kupfer(II)-chlorid-dihydrat in 15 ml Methanol hinzugefügt. Man verfährt weiter wie in Beispiel 1 und erhält 2,51 g Etamycin-Kupferkomplex.

| C$_{88}$H$_{122}$CuN$_{16}$O$_{22}$ (1819,53) | | | | | | |
|---|---|---|---|---|---|---|
| ber. | C | 55,8 % | H | 6,9 % | Cu | 3,4 % |
| gef. | | 55,5 % | | 6,9 % | | 3,6 % |

Die berechneten Analysenwerte sind auf einen Wassergehalt (bestimmt nach Karl Fischer) von 4,0 % bezogen.
$\lambda_{max.}$ (CH$_3$OH) = 353 nm

## 3) Etamycin-Kobalt(II)-komplex Et x Co(II)OH

4,40 g (5 mMol) Etamycin und 1,21 g (5 mMol) Kobalt(II)-chlorid-hexahydrat (98 %ig) werden in 75 ml Methanol gelöst und 10 ml 1n-methanolische NaOH hinzugefügt. Man arbeitet wie in Beispiel 1 beschrieben und erhält 4,6 g Etamycin-Kobaltkomplex.
C$_{44}$H$_{62}$CoN$_8$O$_{12}$ (953,93)
ber.
Co 5,8 %
gef.
6,6 %
(bezogen auf 7,0 % Wassergehalt, der nach Karl Fischer bestimmt wurde)
$\lambda_{max.}$ (CH$_3$OH) = 347 nm

## 4) Etamycin-Zn(II)-komplex (Et$_2$ x Zn(II))

Lösungen von 4,40 g (5 mMol) Etamycin in 50 ml Methanol und von 348 mg (2,5 mMol) Zinkchlorid (98 %ig) in 25 ml Methanol werden zusammengegeben und mit 5 ml 1n-methanolischer NaOH unter Rühren versetzt. Man arbeitet wie in Beispiel 1 beschrieben und erhält 4,65 g Etamycin-Zinkkomplex.

| C$_{88}$H$_{122}$N$_{16}$O$_{22}$Zn (1821,35) | | | | | | |
|---|---|---|---|---|---|---|
| ber. | C | 53,8 % | H | 7,1 % | Zn | 3,3 % |
| gef. | | 54,3 % | | 7,1 % | | 3,3 % |

Die berechneten Analysenwerte sind auf einen Wassergehalt (bestimmt nach Karl Fischer) von 7,3 % bezogen.
$\lambda_{max.}$ (CH$_3$OH) = 350 nm

## 5) Etamycin-Eisenkomplex Et$_3$ x Fe(III)

Man löst 5,27 g (6 mMol) Etamycin in 60 ml Methanol, gibt die Lösung von 546 mg (2 mMol) Eisen(III)-chlorid-hexahydrat (99 %ig) in 30 ml Methanol hinzu und versetzt unter Rühren mit 6 ml 1n-methanolischer NaOH. Aufarbeitung wie in Beispiel 1. Man erhält 5,4 g Etamycin-Eisenkomplex.

| $C_{132}H_{183}FeN_{24}O_{33}$ (2689,82) | | | | | | |
|---|---|---|---|---|---|---|
| ber. | C | 57,2 % | H | 7,0 % | Fe | 2,0 % |
| gef. | | 57,5 % | | 7,1 % | | 2,1 % |

Die berechneten Analysenwerte sind auf einen Wassergehalt (bestimmt nach Karl Fischer) von 3,0 % bezogen.

$\lambda_{max1}$ ($CH_3OH$) = 304 nm

$\lambda_{max2}$ ($CH_3OH$) = 340 nm (Schulter)

6) Etamycin-Aluminiumkomplex $Et_3$ x Al(III)

Eine Lösung von 483 mg (2 mMol) Aluminiumchlorid-hexahydrat in 30 ml Methanol wird zu einer Lösung von 5,27g (6 mMol) Etamycin in 60 ml Methanol gegeben. Nach Hinzufügen von 6 ml 1n-methanolischer NaOH wird gemäß Beispiel 1 aufgearbeitet . Man erhält 5,35 g Etamycin-Aluminiumkomplex.

| $C_{132}H_{183}AlN_{24}O_{33}$ (2660,96) | | | | | | |
|---|---|---|---|---|---|---|
| ber. | C | 57,5 % | H | 7,1 % | Al | 1,0 % |
| gef. | | 57,3 % | | 7,2 % | | 1,0 % |

Die berechneten Analysenwerte sind auf einen Wassergehalt (bestimmt nach Karl Fischer) von 3,5 % bezogen.

$\lambda_{max1}$ ($CH_3OH$) = 314 nm

$\lambda_{max2}$ ($CH_3OH$) = 334 nm (Schulter)

7) Etamycin-Kalziumkomplex $Et_2$ x Ca(II)

4,40 g (5 mMol) Etamycin und 553 mg (2,5 mMol) Kalziumchlorid-hexahydrat (99 %ig) werden in 75 ml Methanol gelöst. Man gibt 5 ml 1n-methanolischer NaOH hinzu und arbeitet wie in Beispiel 1 beschrieben. Man erhält 2,9 g Etamycin-Kalziumkomplex.

| $C_{88}H_{122}CaN_{16}O_{22}$ (1796,06) | | | | | | |
|---|---|---|---|---|---|---|
| ber. | C | 56,0 % | H | 7,1 % | Ca | 2,1 % |
| gef. | | 56,5 % | | 7,3 % | | 2,0 % |

Die berechneten Analysenwerte sind auf einen Wassergehalt (bestimmt nach Karl Fischer) von 4,9 % bezogen.

$\lambda_{max1}$ ($CH_3OH$) = 304 nm

$\lambda_{max2}$ ($CH_3OH$) = 345 nm (Schulter)

8) Etamycin-Magnesiumkomplex $Et_2$ x Mg(II)

Die Lösung von 8,79 g (10 mMol) Etamycin und 1,02 g (5 mMol) Magnesiumchlorid-hexahydrat in 150 ml Methanol wird unter Rühren mit 10 ml 1n-methanolischer NaOH versetzt. Das Gemisch wird nach der Methode des Beispieles 1 aufgearbeitet. Man erhält 6,4 g Etamycin-Magnesiumkomplex.

| $C_{88}H_{122}MgN_{16}O_{22}$ (1780,30) | | | | | | |
|---|---|---|---|---|---|---|
| ber. | C | 56,3 % | H | 7,1 % | Mg | 1,3 % |
| gef. | | 56,4 % | | 7,2 % | | 1,3 % |

Die berechneten Analysenwerte sind auf einen Wassergehalt (bestimmt nach Karl Fischer) von 5,2 % bezogen.

$\lambda_{max1}$ ($CH_3OH$) = 312 nm

$\lambda_{max2}$ ($CH_3OH$) = 343 nm

9) Etamycin-Magnesiumkomplex Et x MgOH

1,76 g (2 mMol) Etamycin gelöst in 20 ml Methanol versetzt man nacheinander mit einer Lösung von 407 mg (2 mMol) Magnesiumchlorid-hexahydrat in 16 ml Methanol und mit 4 ml 1n-methanolischer NaOH. Nach der in Beispiel 1 beschriebenen Methode erhält man 1.66 g Etamycin-Magnesiumkomplex.

$C_{44}H_{62}MgN_8O_{12}$ (919,31)

ber.

Mg 2,5 %

gef.

2,6 %

(bezogen auf 4,9 % Wassergehalt der nach Karl Fischer bestimmt wurde)

$\lambda_{max1}$ ($CH_3OH$) = 312 nm

$\lambda_{max2}$ ($CH_3OH$) = 344 nm

10) Etamycin-Mangan (II)-komplex $Et_2$ x Mn(II)

Eine Lösung von 3,52 g (4 mMol) Etamycin und 400 mg (2 mMol) Mangan(II)-chlorid-tetrahydrat in 60 ml Methanol wird mit 4 ml 1n-methanolischer NaOH versetzt. Nach der in Beispiel 1 beschriebenen Methode erhält man 3,4 g Etamycin-Mangankomplex.

| $C_{88}H_{122}MnN_{16}O_{22}$ (1810,92) | | | | | | |
|---|---|---|---|---|---|---|
| ber. | C | 55,1 % | H | 7,0 % | Mn | 2,9 % |
| gef. | | 55,7 % | | 7,1 % | | 2,9 % |

Die berechneten Analysenwerte sind auf einen Wassergehalt (bestimmt nach Karl Fischer) von 5,6 % bezogen.

$\lambda_{max.}$ ($CH_3OH$) = 350 nm

**Biologische Beispiele**

**Beispiel I:**

Ein Fütterungsversuch mit Etamycin-Cu-Komplex gemäß Herstellungsbeispiel 2 wurde mit 120 männlichen Mastküken in 3 Gruppen zu je 40 Küken durchgeführt, die in beheizten Batterien (5 Küken bei 8 Wiederholungen) gehalten wurden. Folgende Präparate und Dosierungen wurden eingesetzt:

| | mg/kg Futter ppm |
|---|---|
| 1. Gruppe: unbehandelte Kontrolle | - |
| 2. Gruppe: Etamycin-Cu-Komplex | 5 |
| 3. Gruppe: Etamycin-Cu-Komplex | 20 |

'Das Grundfutter hatte eine handelsübliche Zusammensetzung mit 13,0 MJ/kg = 3100 kcal/kg und einem Rohproteingehalt von 22 %, bei einem normgerechten Aminosäuren-, Vitamin-, Mineralstoff- und Spurenelementgehalt.

| Zusammensetzung in % | |
|---|---|
| Fischmehl | 6.00 |
| Futterhefe | 2.00 |
| Fett (Rindertalg) | 4.70 |
| Sojaschrot | 24.00 |
| Luzernegrünmehl | 1.00 |
| Mais | 44.89 |
| Weizen | 6.35 |
| Weizennachmehl | 8.50 |
| phosphors. Futterkalk | 1.40 |
| kohlens. Futterkalk | 0.96 |
| Spurenelementmischung | 0.04 |
| Viehsalz | 0.09 |
| Methionin DL | 0.07 |
| | 100.00 |

Der Versuch lief über 6 Wochen. Futter und Trinkwasser wurden nach Belieben angeboten. Die Tiere wurden wöchentlich einzeln gewogen, die Lebendmasse(LM)-Zunahmen errechnet, die Futteraufnahme bestimmt und aus Futteraufnahme : LM-Zunahme der Futteraufwand errechnet. Die Mortalität wurde erfaßt und aus all diesen Parametern die Produktionszahl (PZ) errechnet.

$$PZ = \frac{\text{tägl. LM-Zunahme (g) x Überlebensrate (\%)}}{\text{Futteraufwand x 10}}$$

Beispiel I

(6 Wochen)

| | Dosis mg/kg Futter ppm | LM-Zunahme | | Futteraufnahme | |
|---|---|---|---|---|---|
| | | in g | rel.% | in g | rel.% |
| Kontrolle | - | 1320 | 100 | 2531 | 100 |
| Etamycin-Cu-Komplex | 5 | 1439 * | 109,0 | 2561 | 101,2 |
| | 20 | 1442 * | 109.2 | 2644 | 104,5 |
| | Futteraufwand | rel. % | Mortalität % | Produktionszahl | |
| Kontrolle | 1,92 | 100 | 10 | 147 | |
| Etamycin-Cu-Komplex | 1,78 | 92,7 | 20 | 154 | |
| | 1,83 | 95,3 | 7,5 | 173 | |

* P 0,05 (P = Irrtumswahrscheinlichkeit)

8

**Beispiel II**

In einem Mastküken-Versuch über 42 Tage wurde mit 144 männlichen Mastküken (Lohmann) 3 x 48 Küken (8 Wiederholungen x 6 Tiere) der Einfluß von Etamycin-Cu-Komplex gemäß Beispiel I in Dosierungen von 5 und 10 ppm gegenüber Negativ-Kontrollen ohne Zusatz geprüft. Haltung und Fütterung waren die gleichen wie in Beispiel I.

Folgende Gruppen wurden eingesetzt:

|  | mg/kg Futter (ppm) |
|---|---|
| 1. Gruppe: unbehandelte Kontrolle | - |
| 2. Gruppe: Etamycin-Cu-Komplex | 5 |
| 3. Gruppe: Etamycin-Cu-Komplex | 10 |

|  | Dosis mg/kg Futter ppm | LM-Zunahme | | Futteraufnahme | |
|---|---|---|---|---|---|
|  |  | in g | rel.% | in g | rel.% |
| Kontrolle | - | 1559 | 100 | 3068 | 100 |
| Etamycin-Cu-Komplex | 5 | 1699 * | 109,0 | 3260 | 106,3 |
|  | 10 | 1673 * | 107,3 | 3099 | 101,0 |
|  | Futteraufwand | rel. % | Mortalität % | Produktionszahl |  |
| Kontrolle | 1,97 | 100 | 8,3 | 173 |  |
| Etamycin-Cu-Komplex | 1,92 | 97,5 | 2,1 | 207 |  |
|  | 1,85 | 93,9 | 6,3 | 202 |  |

* P 0,05

**Beispiel III:**

In einem weiteren Mastküken-Versuch wurde der Einfluß von Etamycin-Zn-Komplex gemäß Beispiel 4 in einer Dosierung von 10 ppm im Vergleich zu einer Negativ-Kontrolle wie im Beispiel I geprüft. Zum Einsatz kamen 36 Mastküken (6 Wiederholungen x 6 Tiere), bei einer Versuchszeit von 6 Wochen. Haltungs- und Fütterungsbedingungen waren die gleichen wie im Beispiel I.

Folgende Gruppen wurden eingesetzt:

|  | mg/kg Futter (ppm) |
|---|---|
| 1. Gruppe: unbehandelte Kontrolle | - |
| 2. Gruppe: Etamycin-Zn-Komplex | 10 |

9

|  | Dosis mg/kg Futter ppm | LM-Zunahme | | Futteraufnahme | |
|---|---|---|---|---|---|
|  |  | in g | rel.% | in g | rel.% |
| Kontrolle | - | 1598 | 100 | 2966 | 100 |
| Etamycin-Zn-Komplex | 10 | 1748 * | 109,4 | 3041 | 102.5 |
|  | Futteraufwand | rel. % | Mortalität % | Produktionszahl |  |
| Kontrolle | 1,86 | 100 | 2,8 | 199 |  |
| Etamycin-Zn-Komplex | 1,74 | 93,5 | 5,6 | 228 |  |

* P 0,05

**Beispiel IV:**

Mit 96 männlichen Mastküken wurde in 3 Gruppen zu je 32 Mastküken (4 Wiederholungen x 8 Tiere) über 5 Wochen der Einfluß des Etamycin-Mn-Komplexes gemäß Beispiel 10 in Dosierungen von 5 und 10 ppm im Vergleich zu einer Negativ-Kontrolle (ohne Zusatz) geprüft. Die Haltungs- und Fütterungsbedingungen sowie die Auswertung waren die gleichen wie in Beispiel I.

Folgende Gruppen wurden eingesetzt:

|  | mg/kg Futter (ppm) |
|---|---|
| 1. Gruppe: unbehandelte Kontrolle | - |
| 2. Gruppe: Etamycin-Mn-Komplex | 5 |
| 3. Gruppe: Etamycin-Mn-Komplex | 10 |

| Ergebnisse |  |  |  |  |  |
|---|---|---|---|---|---|
|  | Dosis mg/kg Futter ppm | LM-Zunahme | | Futteraufnahme | |
|  |  | in g | rel.% | in g | rel.% |
| Kontrolle | - | 1310 | 100 | 2334 | 100 |
| Etamycin-Mn-Komplex | 5 | 1394 * | 106,4 | 2334 | 100 |
|  | 10 | 1419 * | 108,3 | 2413 | 103,4 |
|  | Futteraufwand | rel. % | Mortalität % | Produktionszahl |  |
| Kontrolle | 1,78 | 100 | 6,3 | 204 |  |
| Etamycin-Mn-Komplex | 1,67 | 93,8 | 0 | 222 |  |
|  | 1,70 | 95,5 | 0 | 222 |  |

* P 0,05

**Ansprüche**

1. Streptogramin B-Metallkomplexe der allgemeinen Formel

$$a\,[SB] \times Me^{n+} \times (n-a)\,OH \qquad II$$

worin SB ein Streptogramin B-Antibiotikum
Me ein physiologisch verträgliches Metallkation
n 2 oder 3 und
a eine Zahl von 1 bis n

bedeutet.

2. Metallkomplexe gemäß Anspruch 1

a [Et] x $Me^{n+}$ x (n - a) OH

worin SB Etamycin ( = Neoviridogrisein IV) bedeutet.

3. Streptogramin B- Metallkomplexe gemäß Anspruch 1 oder 2, dadurch gekennzeichnet, daß "Me" Kupfer, Zink, Mangan, Eisen, Kobalt, Magnesium, Kalzium oder Aluminium bedeutet.

4. Verfahren zur Herstellung von Streptogramin B-Metallkomplexen gemäß Anspruch 1 oder 2, dadurch gekennzeichnet, daß man ein Streptogramin B-Antibiotikum und ein physiologisch verträgliches Metallsalz in gelöster Form aufeinander einwirken läßt, mit einer anorganischen Base neutralisiert und aus dem Reaktionsgemisch den Komplex nach an sich bekannten Aufarbeitungsmethoden isoliert.

5. Futterzusatz, gekennzeichnet durch einen Gehalt an einem Streptogramin B-Metallkomplex gemäß Anspruch 1.

6. Futterzusatz, gekennzeichnet durch einen Gehalt an einem Etamycin-Metallkomplex gemäß Anspruch 2.

7. Futterzusatz gemäß Anspruch 5, dadurch gekennzeichnet, daß er aus einem Gemisch verschiedener Streptrogramin B-Metallkomplexe der Formel II besteht.

8. Verwendung von Streptogramin B-Metallkomplexen gemäß Anspruch 1 als Zusatz zu Futtermitteln.

9. Verwendung von Etamycin-Metallkomplexen gemäß Anspruch 2 als Zusatz zu Futtermitteln.

10. Verfahren zur Verbesserung des Wachstums und der Futterverwertung von Nutztieren, dadurch gekennzeichnet, daß man dem Tierfutter 1-100 ppm eines Metallkomplexes gemäß Anspruch 1 oder 2 zumischt.

Patentansprüche für die folgenden Vertragstaaten: ES, GR

1. Futterzusatzmittel, gekennzeichnet durch einen Gehalt an einem Metallkomplex der allgemeinen Formel

a [SB] x $Me^{n+}$ x (n-a) OH      II

worin SB ein Streptogramin B-Antibiotikum
Me ein physiologisch verträgliches Metallkation
n 2 oder 3 und
a eine Zahl von 1 bis n

bedeutet.

2. Futterzusatzmittel gemäß Anspruch 1, dadurch gekennzeichnet, daß die Verbindung der Formel II ein Etamycin ( = Neoviridogrisein IV)-Metallkomplex ist.

3. Futterzusatzmittel gemäß Anspruch 1 oder 2, dadurch gekennzeichnet, daß "Me" in Formel II Kupfer, Zink, Mangan, Eisen, Kobalt, Magnesium, Kalzium oder Aluminium bedeutet.

4. Futterzusatzmittel gemäß Anspruch 2, dadurch gekennzeichnet, daß es sich um den Etamycin-Cu-Komplex handelt.

5. Futterzusatzmittel gemäß Anspruch 2, dadurch gekennzeichnet, daß es sich um den Etamycin-Zn-Komplex handelt.

6. Futterzusatzmittel gemäß Anspruch 2, dadurch gekennzeichnet, daß es sich um den Etamycin-Mn-Komplex handelt.

7. Futterzusatzmittel gemäß Anspruch 1, dadurch gekennzeichnet, daß es aus einem Gemisch verschiedener Streptogramin B-Metallkomplexe besteht.

8. Verwendung von Streptogramin B-Metallkomplexen als Zusatz zu Futtermitteln.

9. Verfahren zur Verbesserung des Wachstums und der Futterverwertung von Nutztieren, dadurch gekennzeichnet, daß man dem Tierfutter 1-100 ppm eines Metallkomplexes der Formel II zumischt.

10. Verfahren zur Herstellung von Streptogramin-B-Metallkomplexen der Formel II, dadurch gekennzeichnet, daß man ein Streptogramin B-Antibiotikum und ein physiologisch verträgliches Metallsaiz in gelöster Form aufeinander einwirken läßt, mit einer anorganischen Base neutralisiert und aus dem Reaktionsgemisch den Komplex nach an sich bekannten Aufarbeitungsmethoden isoliert.